# EUROPEAN PATENT SPECIFICATION

(11) **EP 3 045 531 B1**
(45) Date of publication and mention of the grant of the patent: **30.08.2023**
(21) Application number: 14844430.0
(22) Date of filing: 05.09.2014
(51) Int. Cl.: C12N 5/10, C12M 1/26, C12Q 1/68

(54) **METHOD FOR INDUCING DIFFERENTIATION OF INDUCED PLURIPOTENT STEM CELLS AND METHOD FOR SELECTING INDUCED PLURIPOTENT STEM CELLS**
VERFAHREN ZUR INDUKTION DER DIFFERENZIERUNG INDUZIERTER PLURIPOTENTER STAMMZELLEN UND VERFAHREN ZUR AUSWAHL INDUZIERTER PLURIPOTENTER STAMMZELLEN
PROCÉDÉ PERMETTANT D'INDUIRE LA DIFFÉRENCIATION DE CELLULES SOUCHES PLURIPOTENTES INDUITES ET PROCÉDÉ PERMETTANT DE SÉLECTIONNER DES CELLULES SOUCHES PLURIPOTENTES INDUITES

(30) Priority: 12.09.2013 JP 2013189221
(43) Date of publication of application: 20.07.2016
(73) Proprietor: Kaneka Corporation, Osaka-shi, Osaka 530-8288 (JP); Satomura, Kazuhito, Tokushima-shi, Tokushima 770-8073 (JP)
(72) Inventor: SATOMURA Kazuhito, Tokushima-shi Tokushima 770-8073 (JP)
(74) Representative: Hoffmann Eitle
(86) International application number: PCT/JP2014/073511
(87) International publication number: WO 2015/037535

(56) References cited:
- WO-A1-2012/043814
- WO-A1-2013/047639
- JP-A- 2005 520 516
- TAKANORI TAKEBE ET AL: "Vascularized and functional human liver from an iPSC-derived organ bud transplant", 20130101, 3 July 2013 (2013-07-03), pages 1-5, XP007922049, DOI: 10.1038/NATURE12271IMAGE [retrieved on 2013-07-03]
- TAKANORI TAKEBE ET AL: "Generation of a vascularized and functional human liver from an iPSC-derived organ bud transplant", NATURE PROTOCOLS, vol. 9, no. 2, 23 January 2014 (2014-01-23), pages 396-409, XP055166485, ISSN: 1754-2189, DOI: 10.1038/nprot.2014.020
- MASANORI TAKENAGA: 'Suizo tono Kyobaiyo ni yoru Mouse ES Saibo no Naihaiyo eno Bunka Yudo' JOURNAL OF JAPAN SURGICAL SOCIETY vol. 106, 05 April 2005, page 284, XP008183147
- NATHANIEL S. ET AL.: 'Derivation of Chondrogenically-Committed Cells from Human Embryonic Cells for Cartilage Tissue Regeneration' PLOS ONE vol. 3, no. 6, 25 June 2008, page E2498, XP055084312
- EDITED BY SHIN'YA YAMANAKA ET AL. ES SAIBO KARA NO BUNKA 20 October 2012, SAISEI IRYO SOSHO 1 KANSAIBO, pages 21 - 29, XP008183198
- Stephen F. Badylak ET AL: "Whole-Organ Tissue Engineering: Decellularization and Recellularization of Three-Dimensional Matrix Scaffolds", Annual Review of Biomedical Engineering, vol. 13, no. 1, 15 August 2011 (2011-08-15), pages 27-53, XP055127197, ISSN: 1523-9829, DOI: 10.1146/annurev-bioeng-071910-124743
- Jessica A. Dequach ET AL: "Decellularized Porcine Brain Matrix for Cell Culture and Tissue Engineering Scaffolds", Tissue Engineering Part A, vol. 17, no. 21-22, 1 November 2011 (2011-11-01), pages 2583-2592, XP055489119, ISSN: 1937-3341, DOI: 10.1089/ten.tea.2010.0724

## Description

### Technical Field

The present invention relates to a method for inducing differentiation of induced pluripotent stem cells,

### Background Art

Stem cells play an important role in regenerative medicine. Stem cells that are known to have totipotency include embryonic stem cells (ES cells), embryonic carcinoma stem cells (EC cells), embryonic germ stem cells (EG cells), nuclear transfer ES cells, somatic cell-derived ES cells (ntES cells), and induced pluripotent stem cells (iPS cells). Stem cells that are known to have pluripotency include somatic stem cells, tissue stem cells, and adult stem cells. Among the aforementioned cells, induced pluripotent stem cells (iPS cells) have totipotency and are artificially produced from somatic cells. Thus, since iPS cells do not have either ethical problems regarding destruction of embryos or eggs, or problems regarding incompatibility in transplantation, it is anticipated that the iPS cells will be applied to regenerative medicine.

With regard to a method for inducing differentiation of induced pluripotent stem cells (iPS cells) into desired cells, various types of methods have been reported. For example, induction of differentiation of iPS cells into pancreatic cells, hepatic cells, cardiomyocytes, blood cells, germ cells, nerve cells, and the like has been reported. However, for practical use of regenerative medicine, in which induced pluripotent stem cells (iPS cells) are used, it is necessary to establish a method for highly efficiently inducing differentiation of iPS cells into cells having functions of interest.

Patent Literature 1 describes a method for inducing differentiation of stem cells into a certain cell lineage, which comprises culturing stem cells *in vitro* in the presence of a tissue sample and/or an extracellular medium of the tissue sample under conditions for inducing differentiation of the stem cells into the certain cell lineage, wherein the differentiated stem cells have the same cell type as that of the tissue sample. The stem cells used in Patent Literature 1 are embryonic stem cells (ES cells), and Patent Literature 1 contains no descriptions regarding induced pluripotent stem cells (iPS cells).

WO 2013/047639 A1 describes means for reconstituting tissues and organs having mature functions, and a method of preparing a tissue or an organ, comprising co-culturing an organ cell with a vascular endothelial cell and a mesenchymal cell. Takebe et al. (Nature 499(7459): 481-484, 2013) describe a vascularized and functional human liver from an iPSC-derived organ bud transplant. Badylak et al. (Annu Rev Biomed Eng. 13: 27-53, 2011) relate to decellularization and recellularization of three-dimensional matrix scaffolds during whole-organ tissue engineering. DeQuach et al. (Tissue Eng Part A. 17(21-22): 2583-2592, 2011) relate to decellularized porcine brain matrix for cell culture and tissue engineering scaffolds.

### Prior Art Literatures

### Patent Literature

Patent Literature 1: JP Patent Publication (Kohyo) No. 2005-520516 A

### Summary of Invention

### Object to be Solved by the Invention

A problem regarding the practical use of regenerative medicine, in which induced pluripotent stem cells (iPS cells) are used, is considered to be the establishment of a technique of highly efficiently inducing differentiation of undifferentiated iPS cells into cells having functions of interest. In addition, another problem is considered to be the establishment of a method for evaluating and/or controlling the quality of iPS cells. That is to say, it is an object of the present invention to provide a method for highly efficiently inducing differentiation of iPS cells into cells having functions of interest, and a method for selecting iPS cells having high differentiation induction efficiency for differentiation of the produced iPS cells into cells of interest. It is another object of the present invention to provide a kit for selecting iPS cells, which is used for the aforementioned methods.

### Means for Solving the Object

As a result of intensive studies directed towards achieving the aforementioned objects, the present inventors have found that differentiation of iPS cells into cells of interest can be achieved by culturing the iPS cells on a frozen section of tissue and/or organ that is to be regenerated, thereby completing the present invention.

The present invention is defined by the appendend claims. Also disclosed is the following:

### Advantageous Effects of Invention

According to the present invention, it is possible to highly efficiently induce differentiation of iPS cells into cells having functions of interest. Moreover, according to the present invention, it is also possible to select iPS cells having high differentiation induction efficiency for differentiation of the iPS cells into cells of interest from among the produced iPS cells.

### Brief Description of Drawings

[Figure 1] Figure 1 shows microscopic images of iPS cells cultured on a cover glass (control), on a normal liver section, or on a hepatitis liver section (Day 2 and Day 8 of the culture).
[Figure 2] Figure 2 shows microscopic images of iPS cells cultured on a cover glass (control) or on a normal liver section (Day 3 and Day 9 of the culture).
[Figure 3] Figure 3 shows microscopic images of iPS cells cultured on a brain section or on a spinal cord section (Day 3 and Day 9 of the culture).
[Figure 4] Figure 4 shows the results obtained by analyzing the expression of hepatic cell-related genes (AFP, AAT, and ALB) by RT-PCR.
[Figure 5] Figure 5 shows the results obtained by analyzing the expression of nerve cell-related genes (Nestin, MBP, CNPase, and GFAP) by RT-PCR.
[Figure 6] Figure 6 shows the results obtained by immunocytochemically analyzing the expression of AFP.
[Figure 7] Figure 7 shows the results obtained by measuring the ratio of AFP-positive cells in nucleated cells.
[Figure 8] Figure 8 shows the results obtained by immunocytochemically analyzing the expression ofAAT.
[Figure 9] Figure 9 shows the results obtained by measuring the ratio of AAT-positive cells in nucleated cells.
[Figure 10] Figure 10 shows the results obtained by immunocytochemically analyzing the expression of GFAP.
[Figure 11] Figure 11 shows the results obtained by measuring the ratio of GFAP-positive cells in nucleated cells.
[Figure 12] Figure 12 shows the results obtained by immunocytochemically analyzing the expression of CNPase.
[Figure 13] Figure 13 shows the results obtained by immunocytochemically analyzing the expression of CNPase.
[Figure 14] Figure 14 shows the results obtained by measuring the ratio of CNPase-positive cells in nucleated cells.

### Embodiments for Carrying out the Invention

Hereinafter, the present invention will be described more in detail.
(1) Method for inducing differentiation of induced pluripotent stem cells, and method for producing differentiation-induced cells

The present invention relates to a method as defined in the claims.

Examples of a cell marker for hepatic cells include, but are not limited to, α-fetoprotein (AFP), α-1 antitrypsin (AAT), albumin (ALB), tyrosine aminotransferase (TAT), tryptophan 2,3 dioxygenase (TDO2), and cytochrome P450.

Examples of a marker for nerve cells include, but are not limited to, nestin, myelin basic protein (MBP), cyclic nucleotide phosphodiesterase (CNPase), glial fibrillary acidic protein (GFAP), and neurofilament.

Examples of a marker for osteoblasts include, but are not limited to, alkaline phosphatase (ALP), osteopontin, and osteocalcin.

Examples of a marker for pancreatic cells include, but are not limited to, Pdx1, amylase, and carboxypeptidase.

Examples of a marker for chondrocytes include, but are not limited to, Sox9, type II collagen, and aggrecan.

Examples of a marker for cardiomyocytes include, but are not limited to, cardiac troponin I (cTnI), α-myosin heavy chain (a-MHC), α-cardiac actin, and homeobox protein Nkx-2.5.

The form of the "structure comprising cells used in the present invention is defined in claim 1. Taking into consideration the fact that induced pluripotent stem cells (iPS cells) are cultured on this structure, the structure is preferably a sheet-like structure. The culture substrate is coated with a component derived from biological tissues or organ sections. The thickness of the sheet-like structure comprising cells (preferably, a section of biological tissue or organ) is not particularly limited. It is generally about 1 to 100 µm, preferably about 2 to 50 µm, and more preferably about 2 to 20 µm.

The form of the "culture substrate" used in the present invention is not particularly limited. It is preferably a film, a plate or a cover glass.

A tissue or organ section can be preferably collected from a mammal (preferably, a mouse, a human, etc.). In the case of using a biological tissue or organ section, the type of the tissue or organ is not particularly limited, and a tissue or organ section comprising cells of the same cell type as that of differentiated cells which are differentiated from induced pluripotent stem cells (iPS cells), may be used. Examples of such a tissue or organ include, but are not limited to, liver, brain, spinal cord, heart, respiratory organ, reproductive organ, kidney, pancreas, skin, muscle, and skeletal organ. For example, when iPS cells are induced to differentiate into hepatic cells, a liver section may be used, and when iPS cells are induced to differentiate into nerve cells, a section comprising nerve cells (for example, a brain section, a spinal cord section, etc.) may be used.

According to the method of the present invention, induced pluripotent stem cells are induced to differentiate into certain cell lineage, and preferably into a cell lineage, namely nerve

Induced pluripotent stem cells have been established for the first time by introducing four factors Oct3/4, Sox2, Klf4 and c-Myc into mouse fibroblasts, and the established cells have been named as "iPS cells" (Takahashi K, Yamanaka S., Cell, (2006) 126: 663-676). Subsequently, human iPS cells have also been established using the same four factors as described above (Takahashi K, Yamanaka S., et al., Cell, (2007) 131: 861-872.). Moreover, a method of using three factors excluding o-Myc has also been reported (Nakagawa M, Yamanaka S., et al., Nature Biotechnology, (2008) 26, 101-106). Furthermore, it has also been reported that four genes OCT3/4, SOX2, NANOG and LIN28 are introduced into human fibroblasts, so as to establish induced pluripotent stem cells (Yu J., Thomson JA, et al., Science (2007) 318: 1917-1920.). Further, it has also been reported that six genes OCT3/4, SOX2, KLF4, C-MYC, hTERT and SV40 large T are introduced into skin cells to establish induced pluripotent stem cells (Park IH, Daley GQ, et al., Nature (2007) 451: 141-146), and that Oct3/4, Sox2, Klf4, c-Myc, and the like are introduced, not into somatic cells, but into undifferentiated stem cells that are present in postnatal tissues, so as to establish induced pluripotent stem cells (JP Patent Publication (Kokai) No. 2008-307007 A).

As described above, induced pluripotent stem cells (iPS cells) mean cells having multipotency and self-replication ability, which are induced by reprogramming somatic cells or undifferentiated stem cells. The origin of somatic cells is not limited, and the somatic cells may be derived from any one of an non-human embryo, a fetus, and an adult An animal species, from which somatic cells are derived, is not particularly limited, either. The animal species is preferably a mammal, and more preferably a human, a mouse, or the like. Examples of the somatic cells include, but are not limited to, fibroblasts, epithelial cells, hepatic cells, and blood cells. The method for producing induced pluripotent stem cells used in the present invention is not particularly limited, and a factor to be introduced, an introduction method, and the like are not particularly limited, either. In addition to the above-mentioned publications, examples of known publications regarding induced pluripotent stem cells include JP Patent Publication (Kokai) No. 2008-283972 A, US 2008-2336610, US 2009-047263, WO 2007/069666, WO2008/118220, WO 2008/124133, WO 2008/151058, WO 2009/057831, WO 2009/006997, and WO 2009/007852.

In the present invention, after iPS cells have been cultured on feeder cells according to an ordinary method, the feeder cells are removed, and iPS cells are recovered. The recovered iPS cells are suspended in a suitable medium (for example, a Dulbecco's modified Eagle's medium (DMEM) containing fetal bovine serum, etc.). Subsequently, there is prepared a culture cover glass, on which a structure comprising cells (preferably, a biological tissue or organ section) is placed, and the suspension of iPS cells may be added to this cover glass. After addition of the suspension, the cover glass is left at rest, so that floating iPS cells are allowed to adhere to the structure comprising cells. Thereafter, a suitable medium (DMEM containing 5% FBS, etc.) is added thereto, and a culture may be then carried out according to an ordinary method.

Conditions for culturing induced pluripotent stem cells on a structure comprising cells and/or a component derived from the cells are not particularly limited, as long as differentiation of induced pluripotent stem cells can be induced under the conditions. As a medium, for example, a Dulbecco's modified Eagle's medium (DMEM) containing fetal bovine serum, or a medium used for primate ES cells (ReproCELL Inc.) can be used. Various types of growth factors, cytokines, differentiation-inducing factors and the like, which are used to induce differentiation of induced pluripotent stem cells, may be added to the medium before performing the culture. However, in the present invention, by performing a culture on a structure comprising cells, induction of differentiation of induced pluripotent stem cells can be achieved without adding the aforementioned growth factors or differentiation-inducing factors. Examples of various types of growth factors, cytokines or differentiation-inducing factors, which are used to induce differentiation of induced pluripotent stem cells, include, but are not limited to, activin, bFGF, noggin, nicotinamide, retinoic acid, EGF, and glucocorticoid.

### (2) Method for selecting induced pluripotent stem cells

According to the present invention, induced pluripotent stem cells are cultured as described in the claims, so that induced pluripotent stem cells having high differentiation induction efficiency can be selected. As described above, at present, induced pluripotent stem cells are produced by various methods. It is anticipated that the produced induced pluripotent stem cells are induced to differentiate into desired cells, and they are then applied to regenerative medicine and the like. However, in general, the produced induced pluripotent stem cells comprise both cells having high differentiation induction efficiency for differentiating into desired cells and cells having low differentiation efficiency. Thus, it has been desired to develop a technique of selecting induced pluripotent stem cells having high differentiation induction efficiency for differentiation of the produced induced pluripotent stem cells into desired cells from.

According to the present invention, by culturing induced pluripotent stem cells on a structure comprising cells , differentiation of the induced pluripotent stem cells are induced, and the induced pluripotent stem cells having high differentiation induction efficiency for differentiating into desired cells can be selected. The thus selected induced pluripotent stem cells having high differentiation induction efficiency are preserved as a stock, and at the time of need, the preserved cells can be induced to differentiate into desired cells and can be then used in the site of regenerative medicine.

### Reference-Aspect

Moreover, the structure comprising cells namely as a biological tissue or organ section, can be provided as a kit that is used for selecting induced pluripotent stem cells. The aforementioned kit may also comprise, as appropriate, a medium for culturing induced pluripotent stem cells, a cover glass on which the structure comprising cells is established, and the like, in addition to the structure comprising cells and/or a component derived from the cells.

The present invention will be described in detail in the following examples. However, these examples are not intended to limit the scope of the present invention.

### [Examples]

### (1) Production of frozen section

Normal liver, brain and spinal cord were excised from a 6-week-old male ICR mouse. In addition, 1 mg/kg carbon tetrachloride that had been mixed with olive oil at a mixing ratio of 1 : 4 was intraperitoneally administered to ICR mice, so that drug-induced hepatitis was artificially developed in the mice. From these ICR mice, on 1, 2, 3 and 5 days after administration of carbon tetrachloride, hepatitis liver was excised.

Each organ was embedded and mounted in Tissue-Tek^{R} (Sakura Finethechnical) that was an OCT compound, and it was then immersed in liquid nitrogen to produce a block for production of a frozen section. From each of these blocks, a frozen section with a thickness of 6 µm was produced, and was then placed on a circular culture cover glass (poly L lysine-coated type, Matsunami Glass Ind., Ltd.). After drying, it was washed with a phosphate buffer (Phosphate Buffered Saline; hereinafter abbreviated as "PBS"; pH 7.4) twice to wash the OCT compound off, and the resultant was then used for the culture of iPS cells.

### (2) Culture of iPS cells and induction of differentiation thereof

iPS cells were cultured according to an ordinary method, and feeder cells were then removed using an iPS/ES cell releasing solution (CTK solution, ReproCELL Inc.), followed by washing with PBS. Thereafter, the iPS cells were recovered and were then suspended in a Dulbecco's modified Eagle's medium (hereinafter abbreviated as "DMEM", SIGMA) containing 5% fetal bovine serum (hereinafter abbreviated as "FBS", Biological Industries). The above-described circular culture cover glass, on which a frozen section had been placed, was placed on a dish with a diameter of 35 mm (non-coated type, Matsunami Glass Ind., Ltd.), and the iPS cell suspension was then inoculated thereon. The suspending iPS cells were allowed to adhere to the frozen section by leaving the cells at rest for 1 day after completion of the inoculation, and 2 ml of DMEM containing 5% FBS was then added thereto to carry out a culture. Thereafter, the medium was exchanged with a fresh one once three days, and the culture was carried out for a total of 9 days.

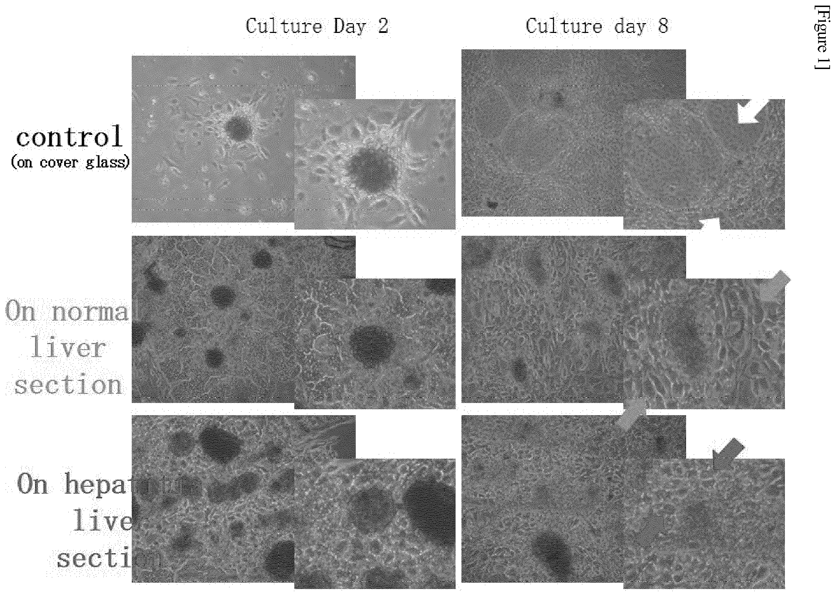

The microscopic images (Day 2 and Day 8 of the culture) of iPS cells cultured on a cover glass (control), on a normal liver section, and on a hepatitis liver section are shown in Figure 1. In all cases of the control, the normal liver, and the hepatitis liver, it was observed that a colony of the inoculated iPS cells extended and the cells were dispersed. Moreover, a change in the shape of cell was also observed, and in the case of the control, when compared with Day 2 of the culture, the extended iPS cells on Day 8 of the culture had various shapes, as shown with the arrow, and no uniformity was found. On the other hand, in the cases of the normal liver and the hepatitis liver, the extended cells tended to have a relatively large and polygonal shape, as shown with the arrow.

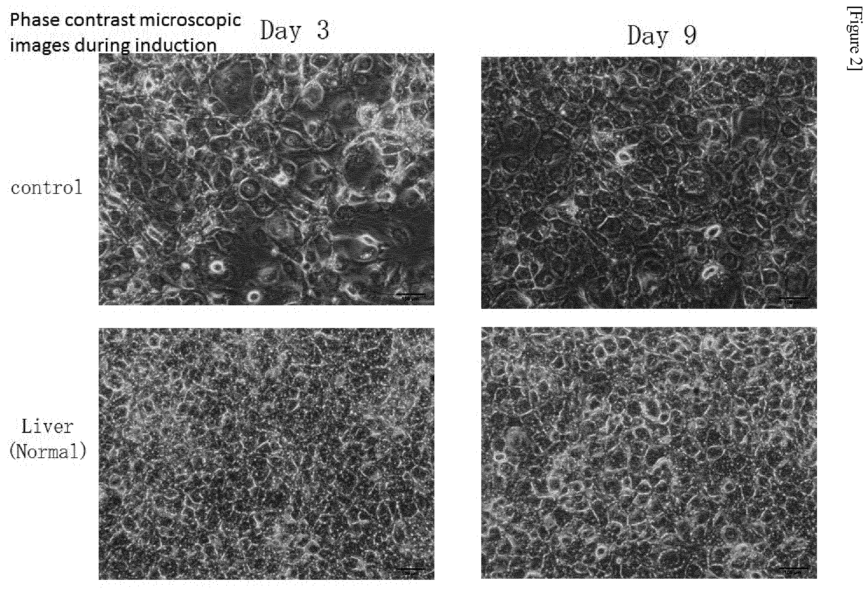

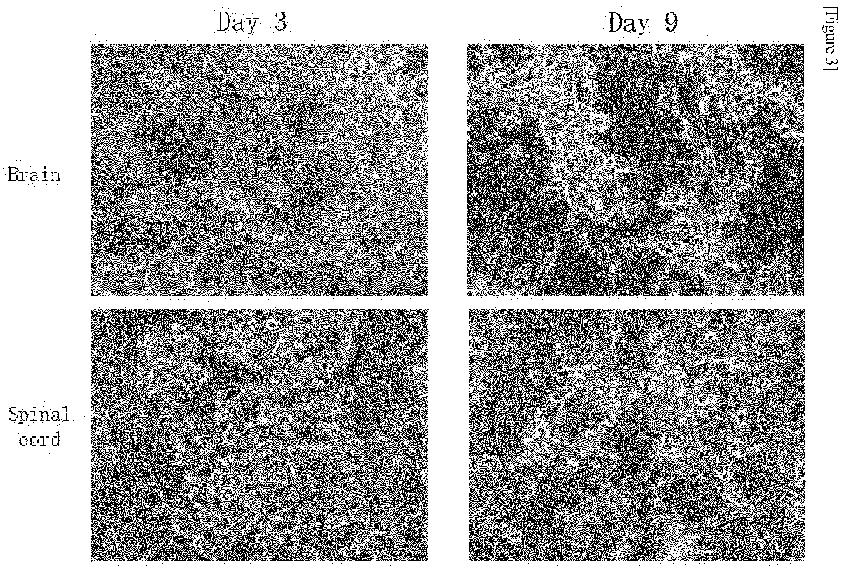

Furthermore, the microscopic images (Day 3 and Day 9 of the culture) of iPS cells cultured on a cover glass (control) and on a normal liver section are shown in Figure 2, and the microscopic images (Day 3 and Day 9 of the culture) of iPS cells cultured on a brain section and on a spinal cord section are shown in Figure 3.

### (3) Confirmation of gene expression

In order to confirm the differentiation state of iPS cells into hepatic cells or nerve cells, the expression of mRNA of various differentiation markers for nerve cells and hepatic cells was analyzed using a semi-quantitative Reverse Transcriptase-Polymerase Chain Reaction method (hereinafter abbreviated as "RT-PCR method"). Total RNA was recovered from iPS cells, which had been cultured on the frozen sections of liver, brain and spinal cord, using TRIzol reagent (Invitrogen Corp., Carlsbad, CA, USA). 1 µg of the obtained total RNA was treated at room temperature with 100 units/ml deoxyribonuclease I (hereinafter abbreviated as "DNase I"), and 1 ml of 25 mM EDTA was then added to the RNA. The obtained mixture was treated at 65°C for 5 minutes, so as to inactivate DNase I. A random hexamer primer (Invitrogen) was added to the reaction mixture, and a reverse transcription reaction was then carried out using reverse transcriptase SUPERSCRIPT III Preamplification System (Invitrogen) under conditions of 50°C and 50 minutes. The reaction mixture was further treated with ribonuclease H at 37°C for 20 minutes, so as to obtain cDNA. Using primers specific to each human gene and PCR MasterMix Kit (Thermo, Rockford, USA), PCR was carried out, and the expression of the mRNA of each gene was then studied. The studied differentiation markers, primers and PCR conditions are shown in Table 1. The PCR product was electrophoresed using 2% agarose gel, was then stained with ethidium bromide, and was then visualized using the UV imaging apparatus FAS-III (TOYOBO Co., Ltd., Osaka).

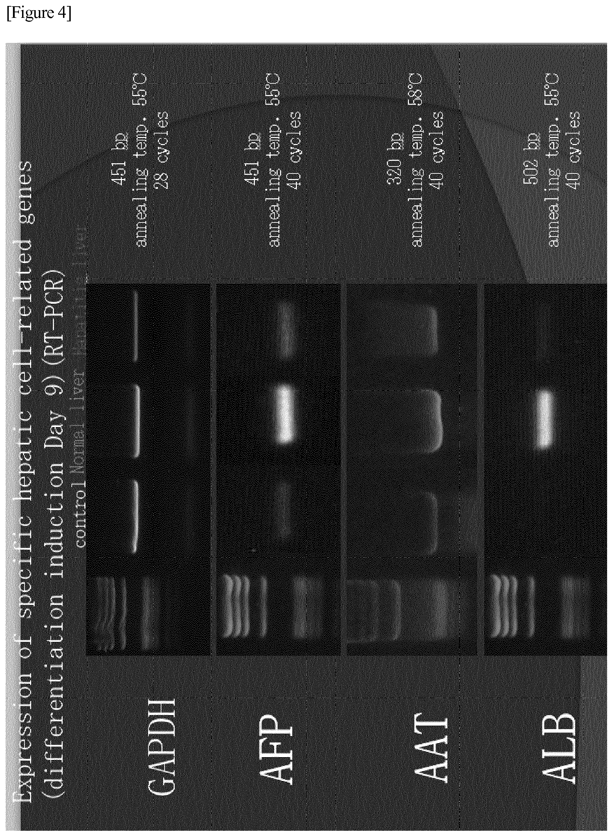

The results obtained by analyzing the expression of hepatic cell-related genes (AFP, AAT, and ALB) are shown in Figure 4.

The expression of AFP was observed in the control group, the normal liver group, and the hepatitis liver group. The expression of AFP tended to be strong in the hepatitis liver group, in comparison to in the control group, and the expression was further increased in the normal liver group. The expression of AAT was also observed in the control group, the normal liver group, and the hepatitis liver group. The expression of AAT was strong in the normal liver group, in comparison to in the control group. On the other hand, the expression of ALB was not observed in the control group, but the expression was confirmed in the normal liver group and the hepatitis liver group. The expression of ALB was strong in the normal liver group, in comparison to in the hepatitis liver group. From these results, it was confirmed that differentiation of iPS cells into specific hepatic cells was induced by culturing the iPS cells on the frozen section of normal liver and hepatitis liver.

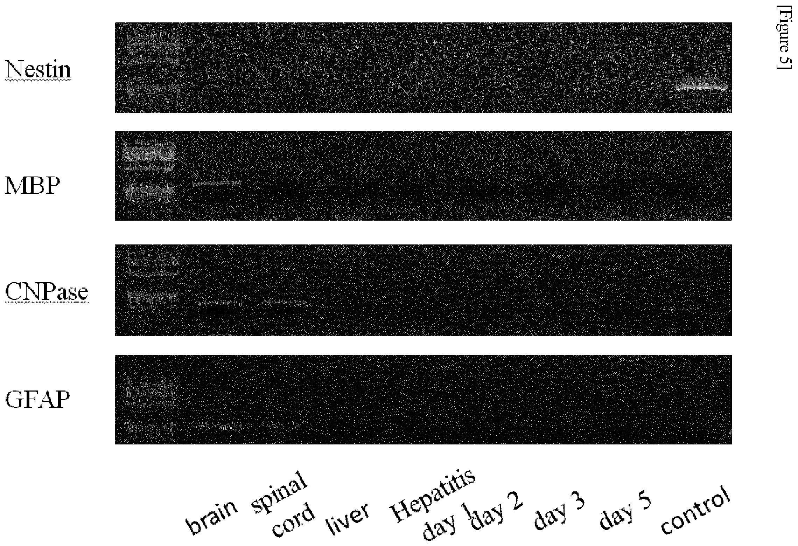

Likewise, the results obtained by analyzing the expression of nerve cell-related genes (Nestin, MBP, CNPase, and GFAP) are shown in Figure 5.

**[Table 1]**

| | Sequence | Size (bp) | Annealing temperature | Number of cycles |
|---|---|---|---|---|
| GAPDH | F: 5'-acc acagtccatgccatc ac-3' (SEQ ID NO: 1) | 451 | 55 | 30 |
| | R: 5'-tcc acc acc ctg ttg ctg ta-3'(SEQ ID NO: 2) | | | |
| AFP | F: 5'-ttt tgg gac ccg aac ttt cc-3' (SEQ m NO: 3) | 451 | 55 | 40 |
| | R: 5'-ctc ctg gtatcc ttt age aac tc-3' (SEQ ID NO: 4) | | | |
| AAT | F: 5'-gca cac cag tec aac agc acc aat-3' (SEQ ID NO: 5) | 320 | 58 | 40 |
| | R: 5'-ccg aag ttg aca gtg aag get tct g-3' (SEQ ID NO: 6) | | | |
| ALB | F: 5'-ggt gtt gat tgc ctt tgc tc-3' (SEQ ID NO: 7) | 502 | 55 | 40 |
| | R: 5'-ccc ttc ate ccg aag ttc at-3' (SEQ ID NO: 8) | | | |
| MBP | F: 5'-ctc cca agg cac aga gac ac-3' (SEQ ID NO: 9) | 207 | 58 | 40 |
| | R: 5'-gga gcc gta gtg agc agt tc-3' (SEQ ID NO: 10) | | | |
| CNPase | F: 5'-cga aaa agc cac aca ttc ct-3' (SEQ ID NO: 11) | 208 | 58 | 40 |
| | R: 5'-cac ggt act tgt cca cga tg-3' (SEQ ID NO: 12) | | | |
| GFAP | F: 5'-aag aga tcc gca cgc agt at-3' (SEQ ID NO: 13) | 392 | 58 | 40 |
| | R: 5'-gta ggt ggc gat etc gat gt-3' (SEQ ID NO: 14) | | | |
| NES | F: 5'-tgc ggg ctactg aaa agt tc-3' (SEQ ID NO: 15) | 311 | 58 | 40 |
| | R: 5'-ggg gag gga agt tgg gct ca-3' (SEQ ID NO: 16) | | | |

| | | | | |
|---|---|---|---|---|
| GAPDH: Glyceraldehyde-3phosphate dehydrogenase AFP: α-Fetoprotein AAT: al- Antitrypsin ALB: Albumin MBP: Myelin basic protein CNPase: 2',3'-Cyclic nucleotide 3'-phosphodiesterase GFAP: Glial fibrillary acidic protein NES: Nestin | | | | |

### (4) Confirmation of protein expression

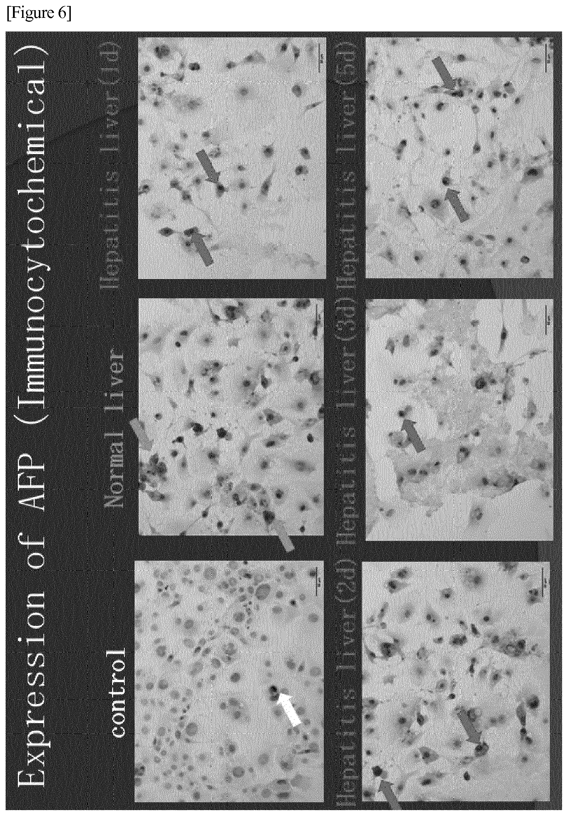

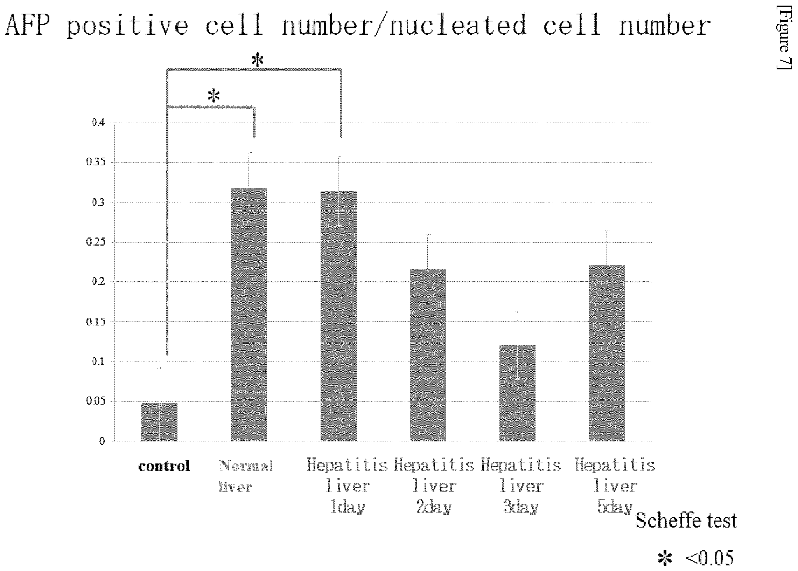

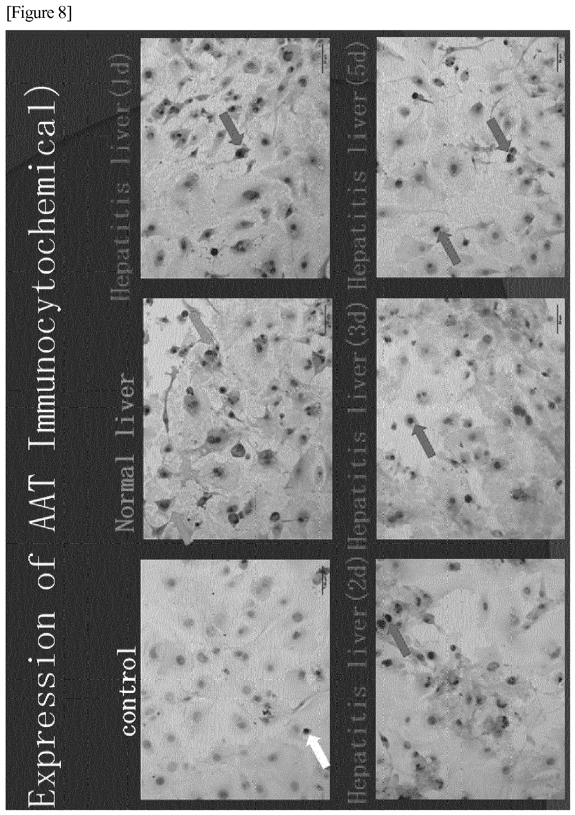

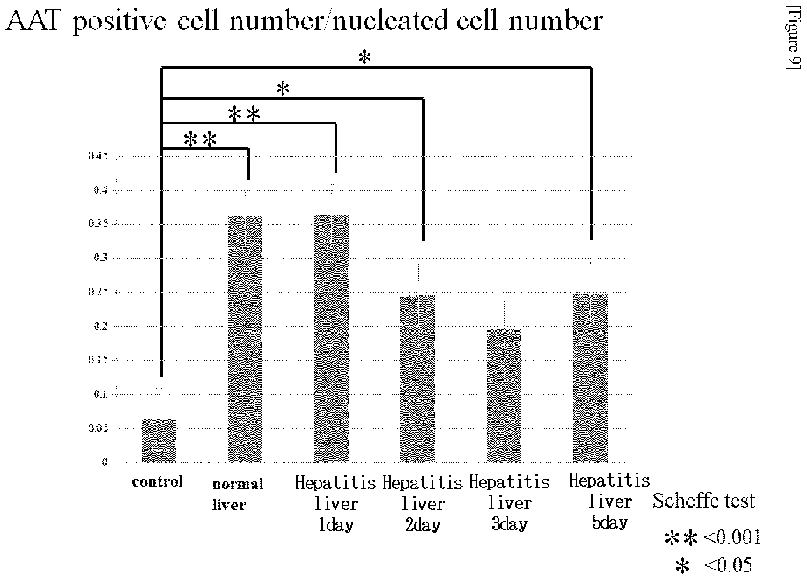

iPS cells cultured on various types of frozen sections were washed with PBS, and the resulting cells, together with the frozen section, were immobilized with 2% paraformaldehyde at room temperature for 60 minutes. The resultant was washed with PBS, was then treated with 1% Triton-X for 15 minutes, and was then blocked with 10% normal rabbit serum or normal goat serum at room temperature for 10 minutes. Thereafter, the resultant was reacted with a rabbit anti-human α-fetoprotein antibody (Abeam, 500 times diluted) or with goat anti-human α1-antitrypsin antibody (Abcam, 300 times diluted) at 4°C overnight. Thereafter, the resultant was washed with PBS, and was then reacted with FITC-conjugated anti-rabbit IgG or FITC-conjugated anti-goat IgG used as a secondary antibody at room temperature for 1 hour. The reaction mixture was washed with PBS, and the nucleus was then stained with DAPI, followed by observation under a fluorescence microscope (Figure 6 and Figure 8). In Figure 6, almost no AFP-positive cells were observed in the control group, but many AFP-positive cells were observed in the normal liver group and in the hepatitis liver group. In Figure 8, as in the case of AFP, many AAT-positive cells were observed in the normal liver group and in the hepatitis liver group, in comparison to in the control group. The number of iPS cells, which expressed α-fetoprotemand α1-antitrypsin, was counted, and the ratio of such iPS cells to the nucleated cells was then calculated (Figure 7 and Figure 9), so that differentiation induction efficiency was analyzed. As shown in Figure 7, a significantly higher ratio of AFP-positive cells was observed in the normal liver group and in the hepatitis liver Day 1 group, than in the control group. Moreover, as shown in Figure 9, a significantly higher ratio of AAT-positive cells was observed in the normal liver group and in the hepatitis liver Day 1, 2 and 5 groups, than in the control group.

Furthermore, using an anti-human GFAP antibody or an anti-CNPase antibody as a primary antibody, the expression of a protein was observed under a fluorescence microscope. iPS cells cultured on various types of frozen sections were immobilized with 2% paraformaldehyde for 1 hour, and were then washed with PBS twice. Thereafter, the resulting cells were treated for 15 minutes with 1% TRITON X-100 (ICN Biomedical) that had been diluted with 0.1% BSA-containing PBS. The resulting cells were washed with PBS twice, and were then blocked with 10% normal goat serum for 1 hour. The resultant was washed with PBS twice, and was then reacted with a primary antibody diluted with 1% BSA-containing PBS at 4°C overnight. The used primary antibody was Rabbit anti-Glial Fibrillary Acidic Protein antibody (SIGMA, 80 times diluted) or Rabbit anti-CNPase antibody (Abeam, 100 times diluted). The reaction mixture was washed with PBS three times, and was then reacted with a secondary antibody diluted with 1% BSA-containing PBS for 1 hour. For GFAP, Goat anti-rabbit IgG FITC (Wako, 40 times diluted) was used as a secondary antibody, and for CNPase, Goat anti-rabbit IgG Alexa (Invitergen, 100 times diluted) was used as a secondary antibody. The resultant was washed with PBS twice, and was then stained with DAPI for 30 minutes. The resultant was washed with PBS twice, was then enclosed with VECTASHIELD, and was then observed under a fluorescence microscope.

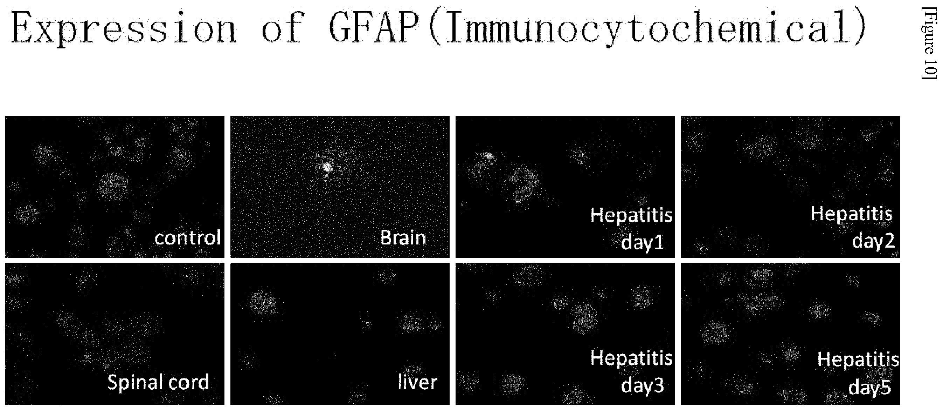

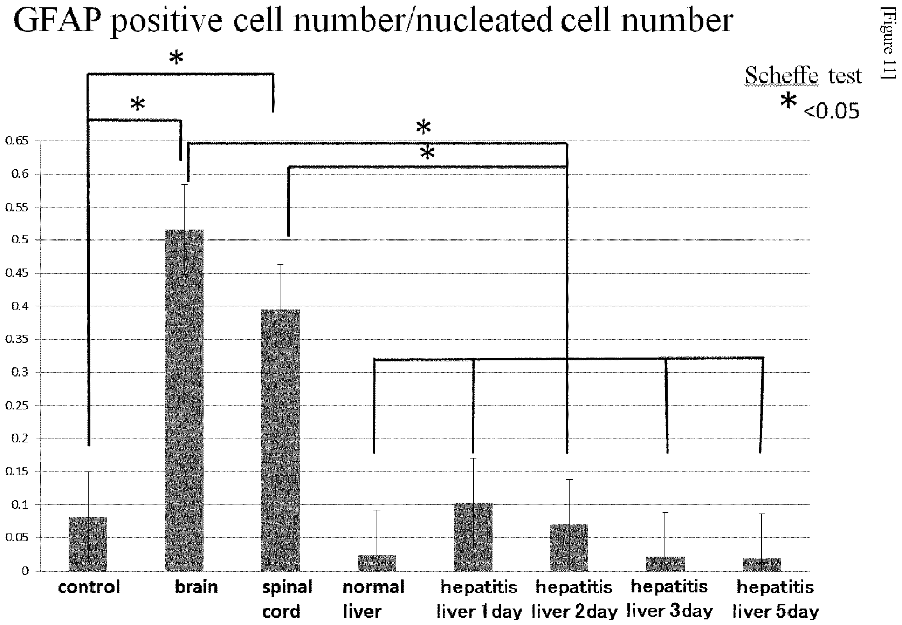

The results obtained by observing under a fluorescence microscope the expression of a protein using an anti-human GFAP antibody as a primary antibody are shown in Figure 10. The number of iPS cells, which expressed GFAP, was counted, and the ratio of such iPS cells to the nucleated cells was then calculated (Figure 11), so that differentiation induction efficiency was analyzed.

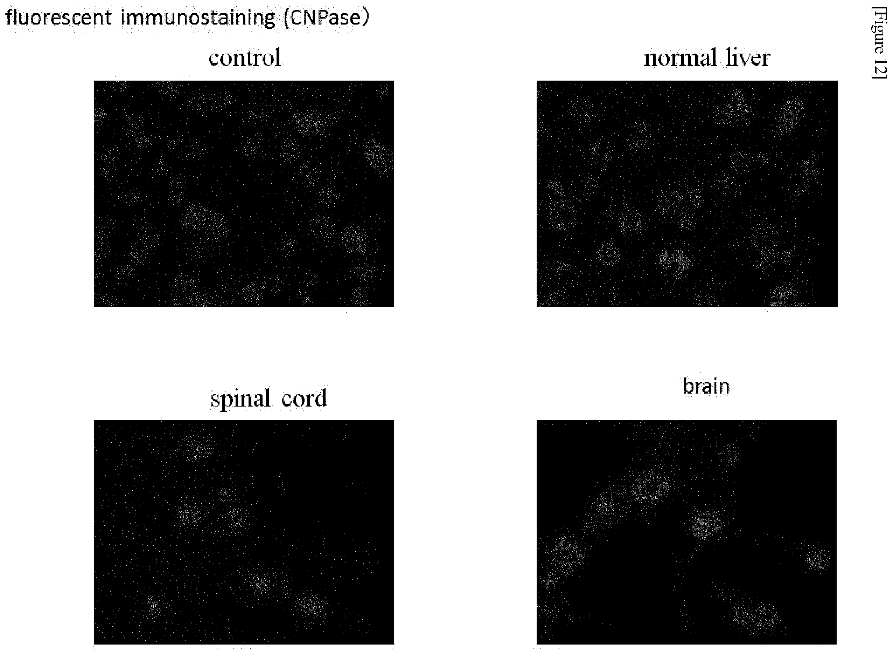

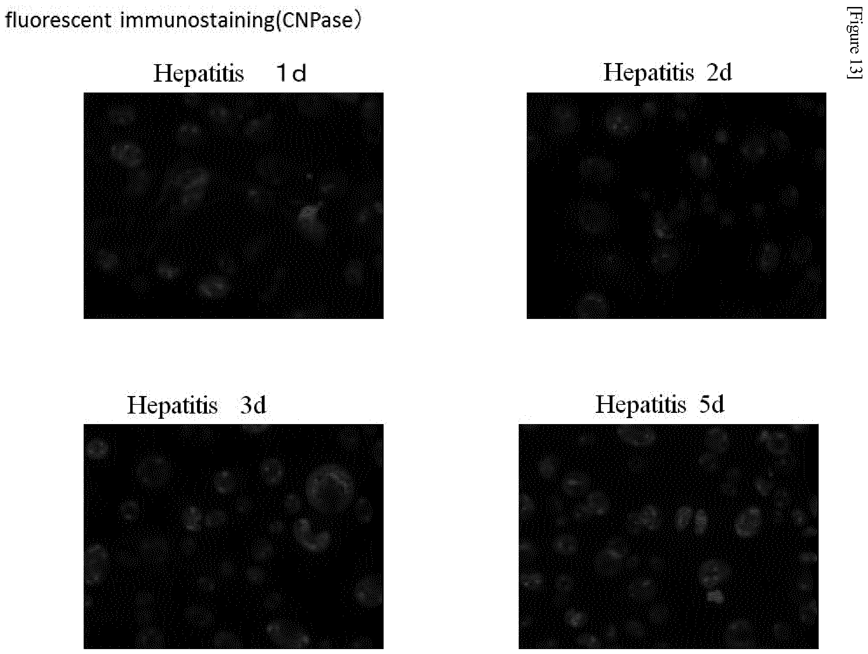

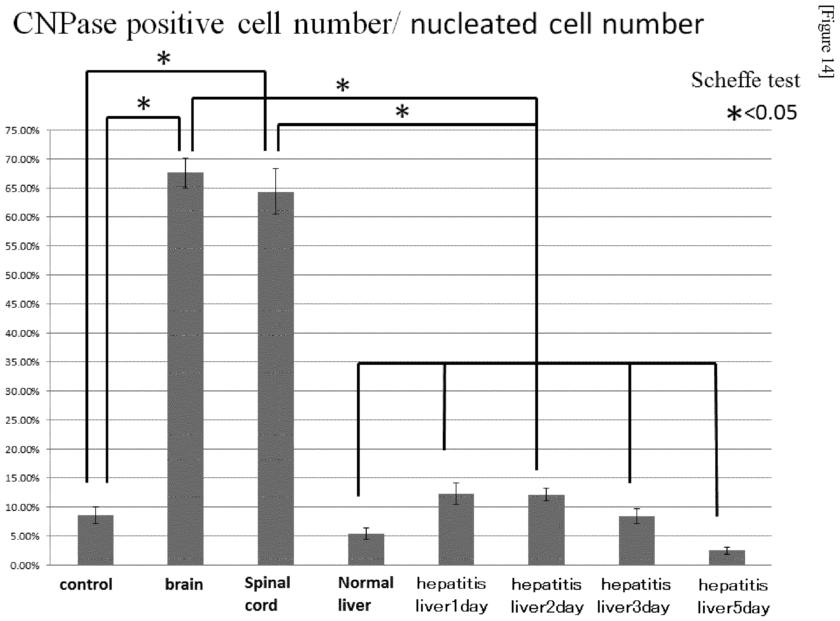

The results obtained by observing under a fluorescence microscope the expression of a protein using an anti-CNPase antibody as a primary antibody are shown in Figure 12 and Figure 13. The number of iPS cells that expressed CNPase was counted, and the ratio of such iPS cells to the nucleated cells was then calculated (Figure 14), so that differentiation induction efficiency was analyzed.

The results regarding cell morphology and the expression of genes (AFP, AAT, and ALB) in a case where iPS cells were cultured on a cover glass (control), on a normal liver section, and on a hepatitis liver section are shown in Table 2. The results regarding the expression of the proteins (AFP and AAT) are shown in Table 3. In addition, the results regarding the expression of GFAP and CNPase genes and proteins are shown in Table 4.

**[Table 2]**

| | Cover glass (control) | Normal liver | Hepatitis liver |
|---|---|---|---|
| Cell morphology | No uniformity found | Large polygonal | Large polygonal |
| AFP | + | +++ | ++ |
| AAT | + | ++ | + |
| ALB | - | ++ | + |

**[Table 3]**

| | Cover glass (control) | Normal liver | Hepatitis liver (Day 1) | Hepatitis liver (Day 2) | Hepatitis liver (Day 3) | Hepatitis liver (Day 5) |
|---|---|---|---|---|---|---|
| AFP | ± | +++ | +++ | ++ | + | ++ |
| AAT | ± | +++ | +++ | ++ | + | ++ |

**[Table 4]**

| | Control | Brain | Spinal cord | Normal liver | Hepatitis liver (Day 1) | Hepatitis liver (Day 2) | Hepatitis liver (Day 3) | Hepatitis liver (Day 5) |
|---|---|---|---|---|---|---|---|---|
| Cell morphology | No uniformity found | Asymmetric, with projections | Asymmetric, with projection | large polygonal | large polygonal | large polygonal | large polygonal | large polygonal |
| GFAP gene expression | + | ++ | ++ | - | - | - | - | - |
| CNPase gene expression | + | +++ | ++ | - | - | - | - | - |
| GFAP protein expression | ± | + | + | ± | ± | 4- | ± | ± |
| CNPase protein expression | ± | + | + | ± | ± | ± | ± | ± |

From the aforementioned results, it was demonstrated that the method of the present invention enables induction of the differentiation of iPS cells into hepatic cells and nerve cells.

## Claims

1. A method for inducing differentiation of induced pluripotent stem cells into differentiated cells, which comprises culturing induced pluripotent stem cells on a structure comprising nerve cells, wherein the cells in the structure and the differentiated cells are the same type of cell, and wherein the structure comprising nerve cells is a culture substrate coated with a section of biological tissue and/or organ.

2. A method according to claim 1, wherein the cells in the structure and the differentiated cells express at least one same marker for the nerve cells.

3. A method according to claim 2, wherein the same marker for the nerve cells is at least one selected from the group consisting of nestin, myelin basic protein, cyclic nucleotide phosphodiesterase, glial fibrillary acidic protein and neurofilament.

4. The method according to claims 1 to 3, wherein the structure comprising nerve cells is a sheet-like structure.

5. The method according to any one of claims 1 to 4, wherein the nerve cells are cells of the brain or spinal cord.

6. A method for producing differentiated nerve cells, which comprises culturing induced pluripotent stem cells on a structure comprising nerve cells, wherein the cells in the structure and the differentiated cells are the same type of cell, and wherein the structure comprising nerve cells is a culture substrate coated with a section of biological tissue and/or organ.

7. A method according to claim 6, wherein the cells in the structure and the differentiated cells express at least one same marker for the nerve cells.

8. A method according to claim 7, wherein the same marker for the nerve cells is at least one selected from the group consisting of nestin, myelin basic protein, cyclic nucleotide phosphodiesterase, glial fibrillary acidic protein and neurofilament.

9. The method according to claim 6 to 8, wherein the structure comprising nerve cells is a sheet-like structure.

10. The method according to any one of claims 6 to 9, wherein the nerve cells are cells of the brain or spinal cord.

## Patentansprüche

1. Verfahren zur Induzierung der Differenzierung von induzierten pluripotenten Stammzellen in differenzierte Zellen, das Kultivieren von induzierten pluripotenten Stammzellen auf einer Struktur umfasst, die Nervenzellen umfasst, wobei die Zellen in der Struktur und die differenzierten Zellen der gleiche Zelltyp sind, und wobei die Struktur, die Nervenzellen umfasst, ein Kultursubstrat überzogen mit einem Abschnitt eines biologischen Gewebes und/oder Organs ist.

2. Verfahren nach Anspruch 1, wobei die Zellen in der Struktur und die differenzierten Zellen mindestens einen gleichen Marker für die Nervenzellen exprimieren.

3. Verfahren nach Anspruch 2, wobei der gleiche Marker für die Nervenzellen mindestens einer ist, der aus der Gruppe bestehend aus Nestin, basischem Myelinprotein (myelin basic protein), zyklischer Nukleotidphosphodiesterase, saurem Gliafaserprotein (glial fibrillary acidic protein) und Neurofilament ausgewählt ist.

4. Verfahren nach einem der Ansprüche 1 bis 3, wobei die Struktur, die Nervenzellen umfasst, eine blattartige Struktur ist.

5. Verfahren nach irgendeinem der Ansprüche 1 bis 4, wobei die Nervenzellen Zellen des Gehirns oder des Rückenmarks sind.

6. Verfahren zur Herstellung differenzierter Nervenzellen, das Kultivieren von induzierten pluripotenten Stammzellen auf einer Struktur umfasst, die Nervenzellen umfasst, wobei die Zellen in der Struktur und die differenzierten Zellen der gleiche Zelltyp sind, und wobei die Struktur, die Nervenzellen umfasst, ein Kultursubstrat überzogen mit einem Abschnitt eines biologischen Gewebes und/oder Organs ist.

7. Verfahren nach Anspruch 6, wobei die Zellen in der Struktur und die differenzierten Zellen mindestens einen gleichen Marker für die Nervenzellen exprimieren.

8. Verfahren nach Anspruch 7, wobei der gleiche Marker für die Nervenzellen mindestens einer ist, der aus der Gruppe bestehend aus Nestin, basischem Myelinprotein (myelin basic protein), zyklischer Nukleotidphosphodiesterase, saurem Gliafaserprotein (glial fibrillary acidic protein) und Neurofilament ausgewählt ist.

9. Verfahren nach einem der Ansprüche 6 bis 8, wobei die Struktur, die Nervenzellen umfasst, eine blattartige Struktur ist.

10. Verfahren nach irgendeinem der Ansprüche 6 bis 9, wobei die Nervenzellen Zellen des Gehirns oder des Rückenmarks sind.

## Revendications

1. Procédé d'induction de la différenciation de cellules-souches pluripotentes induites en cellules différenciées, qui comprend la mise en culture de cellules-souches pluripotentes induites sur une structure comprenant des cellules nerveuses, dans lequel les cellules dans la structure et les cellules différenciées sont le même type de cellule, et dans lequel la structure comprenant des cellules nerveuses est un substrat de culture recouvert d'une section de tissu biologique et/ou d'organe.

2. Procédé selon la revendication 1, dans lequel les cellules dans la structure et les cellules différenciées expriment au moins un même marqueur pour les cellules nerveuses.

3. Procédé selon la revendication 2, dans lequel le même marqueur pour les cellules nerveuses est au moins un sélectionné dans le groupe consistant en une nestine, une protéine basique de myéline, un nucléotide cyclique phosphodiestérase, une protéine acide fibrillaire gliale et un neurofilament.

4. Procédé selon les revendications 1 à 3, dans lequel la structure comprenant des cellules nerveuses est une structure assimilable à une feuille.

5. Procédé selon l'une quelconque des revendications 1 à 4, dans lequel les cellules nerveuses sont des cellules du cerveau ou de la moelle épinière.

6. Procédé de production de cellules nerveuses différenciées, qui comprend la mise en culture de cellules-souches pluripotentes induites sur une structure comprenant des cellules nerveuses, dans lequel les cellules dans la structure et les cellules différenciées sont le même type de cellule, et dans lequel la structure comprenant des cellules nerveuses est un substrat de culture recouvert d'une section de tissu biologique et/ou d'organe.

7. Procédé selon la revendication 6, dans lequel les cellules dans la structure et les cellules différenciées expriment au moins un même marqueur pour les cellules nerveuses.

8. Procédé selon la revendication 7, dans lequel le même marqueur pour les cellules nerveuses est au moins un sélectionné dans le groupe consistant en une nestine, une protéine basique de myéline, un nucléotide cyclique phosphodiestérase, une protéine acide fibrillaire gliale et un neurofilament.

9. Procédé selon les revendications 6 à 8, dans lequel la structure comprenant des cellules nerveuses est une structure assimilable à une feuille.

10. Procédé selon l'une quelconque des revendications 6 à 9, dans lequel les cellules nerveuses sont des cellules du cerveau ou de la moelle épinière.
